# EUROPEAN PATENT SPECIFICATION

(11) **EP 4 009 451 B1**
(45) Date of publication and mention of the grant of the patent: **11.03.2026**
(21) Application number: 21212141.2
(22) Date of filing: 03.12.2021
(51) Int. Cl.: H01R 13/64

(54) **HIGH-DENSITY CONNECTOR**
VERBINDER VON HOHER DICHTE
CONNECTEUR HAUTE DENSITÉ

(30) Priority: 04.12.2020 US 202017112773
(43) Date of publication of application: 08.06.2022
(73) Proprietor: Biosense Webster (Israel) Ltd, Yokneam, 2066717 (IL)
(72) Inventor: BARNEA, Nadav, Yokneam 2066717 (IL); VILENSKY, Alek, Yokneam 2066717 (IL)
(74) Representative: Carpmaels & Ransford LLP

(56) References cited:
- US-A- 3 054 080
- US-A- 4 808 127

## Description

### FIELD OF THE INVENTION

The present invention relates generally to medical devices, and particularly to electrical connectors for these devices.

### BACKGROUND

Medical procedures, such as radio-frequency ablation, electroporation, and electrophysiological measurements within the heart and other internal organs, utilize a catheter inserted in a body of a subject. The catheter, comprising electrodes for both transmitting and receiving electrical signals between the body tissue and control electronics external to the body, is connected by an electrical connector to external electronics.

In US 3054080 A, there are described electrical connectors and, more specifically, receptacles and plugs of the type used on electrical appliances having removable cord and plug sets.

In US 4808127 A, there is described a connector assembly comprising a female connector having a protruding portion extending from one end and with one or more sockets therein and a male connector having one or more pins for being received in a different one of the sockets and a retractable pin shield member in which the pins are recessed when the shield member is in a forward position.

### SUMMARY

The invention is defined by the appended independent claim. Embodiments of the invention are defined in the dependent claims. Embodiments of the present invention that are described hereinbelow provide improved electrical connectors, particularly for medical devices.

There is therefore provided, in accordance with an embodiment of the present invention, an electrical connector assembly. The electrical connector assembly includes a female connector, which includes a female connector housing defining a cavity having an hourglass shape and a first array of electrically-conductive pins disposed within the cavity. The electrical connector assembly further includes a male connector, which includes a male connector housing having an hourglass-shaped protrusion dimensioned to be inserted into and fit tightly within the cavity and a second array of electrically conductive sockets, which are contained within the protrusion and are dimensioned and aligned so that upon insertion of the protrusion into the cavity, each of the pins is introduced into and makes electrical contact with a respective one of the sockets.

The hourglass shape includes peripheral areas on opposing sides of a central area, wherein the peripheral areas are not symmetrical about the central area.

In a further embodiment, the pins are recessed inside the cavity, whereby the pins contact the sockets only after the protrusion has been inserted into the cavity. According to the invention, the first array includes at least 100 pins.

In yet another embodiment, the protrusion includes centering holes having lead-in chamfers that are aligned with the sockets.

There is also provided, in accordance with an embodiment of the present invention, an electrical connector. The electrical connector includes a male connector housing having an hourglass-shaped protrusion dimensioned to be inserted into and fit tightly within an hourglass-shaped cavity of a female connector, which includes a first array of electrically-conductive pins disposed within the cavity. The electrical connector further includes a second array of electrically-conductive sockets, which are contained within the protrusion and are dimensioned and aligned so that upon insertion of the protrusion into the cavity, each of the pins is introduced into and makes electrical contact with a respective one of the sockets.

The present invention will be more fully understood from the following detailed description of the embodiments thereof, taken together with the drawings in which:

### BRIEF DESCRIPTION OF THE DRAWINGS

Fig. 1 is a schematic pictorial illustration of a connector assembly, in accordance with an embodiment of the invention;
Fig. 2 is a schematic frontal view of a male connector in the connector assembly of Fig. 1, in accordance with an embodiment of the invention; and
Fig. 3 is a partial sectional view of the connector assembly of Fig. 1, in accordance with an embodiment of the invention.

### DETAILED DESCRIPTION OF EMBODIMENTS

As the number of electrodes on a catheter increases, the number of connecting conductors (wires or traces) correspondingly increases. These conductors are connected to external electronics so that data from the electrodes can be acquired and also so that signals can be transmitted to the electrodes. For large numbers of conductors, such as for catheters with 100 or more electrodes, there is a need for a connector with a high pin count that can be connected and disconnected repeatedly with precise alignment, is robust, and continues to operate without problems (such as by broken or damaged individual connecting pins) over multiple cycles of connection and disconnection.

The embodiments of the present invention that are described herein address this challenge by providing a connector assembly that combines high mechanical strength with precise alignment. The alignment is facilitated by deeply recessing the conductor pins in a cavity within the housing of the female connector, and constructing the male connector to have a long protrusion that conforms to the shape of the cavity. The male connector has pin-receiving sockets, which align with the pins when the protrusion is inserted into the cavity. The long protrusion and mating body cavity ensure that pins and sockets align exactly before they actually engage, thus virtually eliminating the possibility of bent pins. The recessing of both the pins and the sockets ensures that inadvertent contact with the pins or the pin-receiving sockets does not occur. This design is especially (though not exclusively) well suited for connectors with large numbers of pins, for example one hundred pins or more.

In the disclosed embodiments, the connector assembly comprises a female connector and a male connector. The housing of the female connector defines a cavity with an hourglass-shaped cross-section. The term "hourglass-shaped" is used in the context of the present description and in the claims in its conventional sense and refers to the cross-sectional shape of the cavity (as well as of a protrusion of the male connector). The cross-section comprises peripheral areas on opposing sides of a central area, with the peripheral areas wider than the central area. The female connector has an array of electrically-conducting pins within the cavity, where the pins are recessed within and protected from external forces by the sidewalls of the cavity. The male connector has a protrusion, which is similarly hourglass-shaped, and dimensioned for insertion into the cavity of the female connector with a tight fit. Within the protrusion, the male connector has an array of electrically-conducting sockets. The sockets are dimensioned and aligned so that upon insertion of the protrusion of the male connector into the cavity of the female connector, each of the pins is introduced into and makes electrical contact with a respective socket.

The tight fit of the protrusion of the male connector in the cavity of the female connector assures that the pins and the sockets are precisely aligned even before the pins enter the respective sockets. The hourglass-shape of the housing of the female connector provides high mechanical strength for its sidewalls for protection of the pins. The hourglass-shape, with a suitable asymmetry between the two peripheral areas of the hourglass, also provides a unique orientation for the insertion of the male connector into the female connector, thus avoiding bending or otherwise damaging the pins due to an improper alignment between the pins and the sockets.

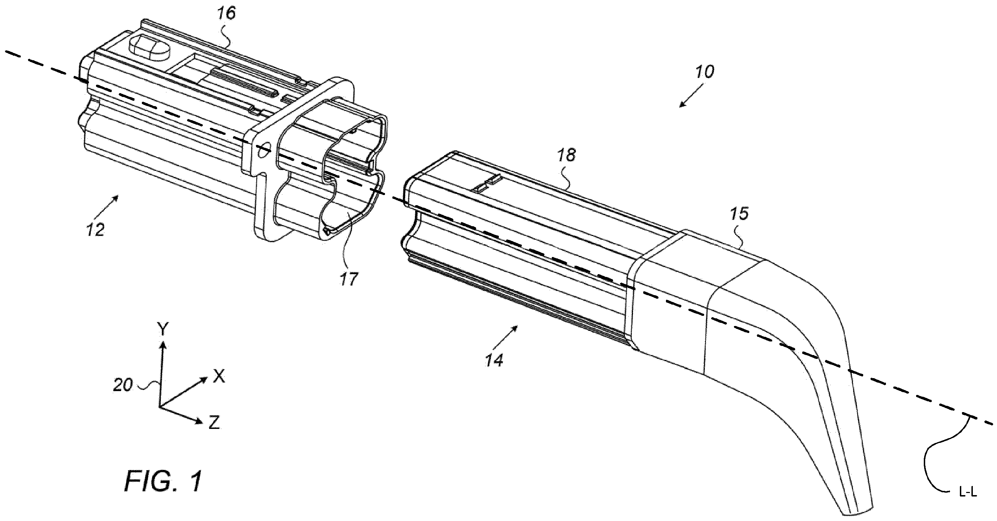

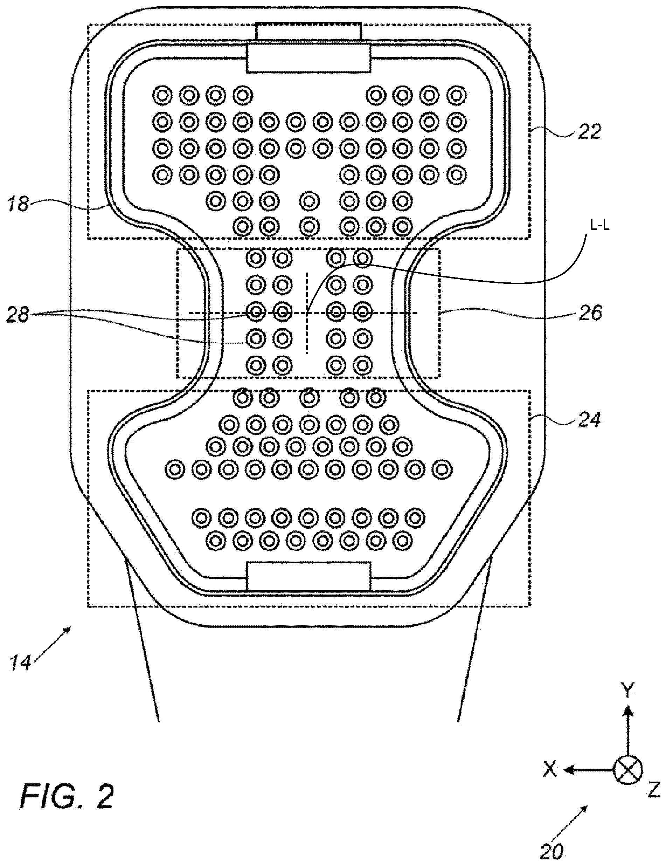

Fig. 1 is a schematic pictorial illustration of a connector assembly 10, in accordance with an embodiment of the invention. Connector assembly 10 comprises a female connector 12 and a male connector 14. Female connector 12 comprises a housing 16 containing a cavity 17 with an hourglass-shaped cross-section (with the hourglass-shape further shown in Fig. 2). Male connector 14 comprises a housing 15 having a protrusion 18, whose cross-section is similarly shaped as an hourglass. Protrusion 18 is dimensioned so that it can be inserted, with a tight fit, into cavity 17 by sliding it in the negative z-direction of Cartesian coordinates 20 which can be referenced to central axis L-L. (For clarity, Cartesian coordinates 20 are also shown in Figs. 2-3 in an appropriate orientation for each figure.)

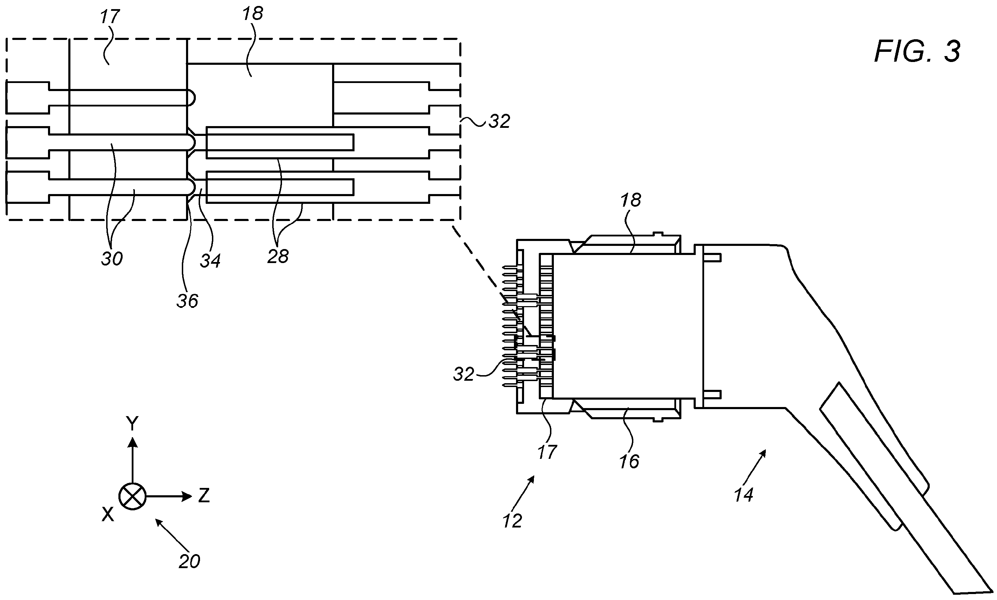

The hourglass shape of cavity 17 provides a mechanically strong structure in order to protect electrically-conducting pins 30 (Fig. 3) located within the cavity. This kind of protection is especially important for connector assemblies with a large number of pins, such as connector assembly 10 with 122 pins (Fig. 2). The tight fit of protrusion 18 within cavity 17 assures a good alignment between pins 30 and electrically-conducting sockets 28 of protrusion 18 (Fig. 2), thus avoiding damage to the pins.

Fig. 2 is a schematic frontal view of male connector 14 of connector assembly 10, in accordance with an embodiment of the invention. In the frontal view (viewed along the z-direction), the hourglass-shape of the cross-section of protrusion 18 is clearly visible, with an upper peripheral area 22, a lower peripheral area 24, and a central area 26, disposed one above another in the Y-direction. The peripheral areas are wider than the central area in their X-dimensions, i.e., in the dimension transverse to the axis along which areas 22, 24 and 26 are disposed. Male connector 14 has an array of 122 electrically-conducting sockets 28 aligned along the Z-direction (coinciding with central axis L-L) within protrusion 18. Sockets 28 connect to 122 electrically-conducting pins 30 (Fig. 3) of female connector 12.

Upper and lower peripheral areas 22 and 24 are not symmetrical about central area, meaning in the pictured example that they do not have reflection symmetry with respect to the X-axis. In the present embodiment, upper peripheral area 22 has a rectangular shape, whereas lower peripheral area 24 has a trapezoidal shape. This asymmetry between the two peripheral areas prevents inserting male connector 14 into female connector 12 in the wrong orientation, and thus prevents misalignment between sockets 28 and pins 30, as such misalignment might cause bending or other damage to the pins.

Fig. 3 is a partial sectional view of connector assembly 10, in accordance with an embodiment of the invention. In Fig. 3, protrusion 18 of male connector 14 has been partially inserted into cavity 17 of female connector 12 to a depth, whereby the outer ends of pins 30 have entered sockets 28. An inset 32 shows the meeting of pins 30 and sockets 28 in greater detail. Pins 30 are recessed inside cavity 17, i.e., the outer ends of the pins are contained inside the cavity, behind the outer plane of housing 16. Thus, pins 30 contact the respective sockets 28 only after the protrusion has been inserted into the cavity. In front of each socket 28, protrusion 18 has a centering hole 34 with a lead-in chamfer 36, aligned with the socket, in order to guide the appropriate pin 30 securely into the socket. In order to effect electrical contacts through connector assembly 10, male connector 14 has to be pushed further into female connector 12, until pins 30 are securely seated inside sockets 28.

Electrical conductors (not shown in the figures) are attached to sockets 28 and pins 30 (for each connector on the side opposite to the other connector) and carry electrical signals to and from connector assembly 10. Both sockets 28 and pins 30 have suitable extensions for attaching these conductors by soldering, pressing, or by other methods known to those skilled in the assembly of electronic components.

## Claims

1. An electrical connector (14), comprising:
a male connector housing (15) having an hourglass-shaped protrusion (18) dimensioned to be inserted into and fit tightly within an hourglass-shaped cavity (17) of a female connector (12), the hourglass-shaped protrusion comprising upper (22) and lower (24) peripheral areas on opposing sides of a central area (26), the upper and lower peripheral areas (24) being wider than the central area (26), the female connector (12) including a first array of electrically-conductive pins (30) disposed within the cavity (17); and
a second array of electrically-conductive sockets (28), which are contained within the protrusion (18) and are dimensioned and aligned so that upon insertion of the protrusion (18) into the cavity (17), each of the pins (30) is introduced into and makes electrical contact with a respective one of the sockets (28), wherein a first socket of the second array is positioned within the upper peripheral area (22), a second socket of the second array is positioned within the lower peripheral area,
**characterized in that** a third socket of the second array is positioned within the central area (26);
the upper peripheral area (22) has a rectangular shape and the lower peripheral area (24) has a trapezoidal shape;
and **in that** the second array comprises at least 100 sockets.

2. The connector according to claim 1, wherein the pins are recessed inside the cavity, and wherein the protrusion is shaped so that the pins contact the sockets only after the protrusion has been inserted into the cavity.

3. The connector according to claim 1, wherein the protrusion comprises centering holes having lead-in chamfers (36) that are aligned with the sockets.

4. An electrical connector (12), comprising:
a female connector housing (16) defining a cavity (17) having an hourglass shape, dimensioned to have an hourglass shaped protrusion (18) of a male connector housing (15) inserted into it and fit tightly within it, the hourglass-shaped cavity (17) comprising upper (22) and lower (24) peripheral areas on opposing sides of a central area (26), the upper (22) and lower (24) peripheral areas being wider than the central area (26), the male connector including a second array of electrically-conductive sockets (28); and
a first array of electrically-conductive pins (30) disposed within the cavity (17), the pins (30) being dimensioned and aligned so that upon insertion of the protrusion (18) into the cavity (17), each of the pins is introduced into and makes electrical contact with a respective one of the sockets, wherein a first pin (30) of the first array is positioned within the upper peripheral area (22), a second pin of the first array is positioned within the lower peripheral area (24),
**characterized in that** a third pin of the first array is positioned within the central area (26);
the upper peripheral area (22) has a rectangular shape and the lower peripheral area (24) has a trapezoidal shape;
and **in that** the first array comprises at least 100 pins.

5. The connector according to claim 4, wherein the pins are recessed inside the cavity, whereby the pins contact the sockets only after the protrusion has been inserted into the cavity.

6. An electrical connector assembly (10), comprising:
an electrical connector (14) according to any one of claims 1 to 3; and
an electrical connector (12) according to claim 4 or claim 5.

## Patentansprüche

1. Elektrischer Verbinder (14), umfassend:
ein männliches Verbindergehäuse (15) mit einem sanduhrförmigen Vorsprung (18), der so dimensioniert ist, dass er in einen sanduhrförmigen Hohlraum (17) eines weiblichen Verbinders (12) eingeführt werden kann und fest in diesen passt, wobei der sanduhrförmige Vorsprung obere (22) und untere (24) Umfangsbereiche auf gegenüberliegenden Seiten eines zentralen Bereichs (26) umfasst, wobei die oberen und unteren Umfangsbereiche (24) breiter sind als der zentrale Bereich (26), wobei der weibliche Verbinder (12) eine erste Anordnung von elektrisch leitfähigen Stiften (30) einschließt, die innerhalb des Hohlraums (17) angeordnet sind; und
eine zweite Anordnung von elektrisch leitfähigen Buchsen (28), die in dem Vorsprung (18) enthalten sind und so dimensioniert und ausgerichtet sind, dass bei Einführung des Vorsprungs (18) in den Hohlraum (17) jeder der Stifte (30) in eine jeweilige der Buchsen (28) eingeführt wird und elektrischen Kontakt mit dieser herstellt,
wobei eine erste Buchse der zweiten Anordnung innerhalb des oberen Umfangsbereichs (22) positioniert ist,
eine zweite Buchse der zweiten Anordnung innerhalb des unteren Umfangsbereichs positioniert ist,
**dadurch gekennzeichnet, dass** eine dritte Buchse der zweiten Anordnung innerhalb des zentralen Bereichs (26) positioniert ist;
der obere Umfangsbereich (22) eine Rechteckform aufweist und der untere Umfangsbereich (24) eine trapezförmige Form aufweist;
und dadurch, dass die zweite Anordnung mindestens 100 Buchsen umfasst.

2. Verbinder nach Anspruch 1, wobei die Stifte innerhalb des Hohlraums vertieft sind, und wobei der Vorsprung so geformt ist, dass die Stifte die Buchsen erst kontaktieren, nachdem der Vorsprung in den Hohlraum eingeführt wurde.

3. Verbinder nach Anspruch 1, wobei der Vorsprung Zentrierlöcher umfasst, die Einführungsschrägen (36) aufweisen, die an den Buchsen ausgerichtet sind.

4. Elektrische Verbinder (12), umfassend:
weibliches Verbindergehäuse (16), das einen Hohlraum (17) mit einer Sanduhrform definiert, der so dimensioniert ist, dass ein sanduhrförmiger Vorsprung (18) eines männlichen Verbindergehäuses (15) in ihn eingeführt und fest in ihm eingepasst wird, wobei der sanduhrförmige Hohlraum (17) obere (22) und untere (24) Umfangsbereiche auf gegenüberliegenden Seiten eines zentralen Bereichs (26) umfasst, wobei die oberen (22) und unteren (24) Umfangsbereiche breiter als der zentrale Bereich (26) sind, wobei der männliche Verbinder eine zweite Anordnung von elektrisch leitfähigen Buchsen (28) einschließt; und
eine erste Anordnung von elektrisch leitfähigen Stiften (30), die innerhalb des Hohlraums (17) angeordnet ist, wobei die Stifte (30) so dimensioniert und ausgerichtet sind, dass bei Einführung des Vorsprungs (18) in den Hohlraum (17) jeder der Stifte in eine jeweilige der Buchsen eingeführt wird und elektrischen Kontakt mit dieser herstellt, wobei ein erster Stift (30) der ersten Anordnung innerhalb des oberen Umfangsbereichs (22) positioniert ist, ein zweiter Stift der ersten Anordnung innerhalb des unteren Umfangsbereichs (24) positioniert ist,
**dadurch gekennzeichnet, dass** ein dritter Stift der ersten Anordnung innerhalb des zentralen Bereichs (26) positioniert ist;
der obere Umfangsbereich (22) eine Rechteckform aufweist und der untere Umfangsbereich (24) eine trapezförmige Form aufweist;
und dadurch, dass die erste Anordnung mindestens 100 Stifte umfasst.

5. Verbinder nach Anspruch 4, wobei die Stifte innerhalb des Hohlraums vertieft sind, wodurch die Stifte die Buchsen erst kontaktieren, nachdem der Vorsprung in den Hohlraum eingeführt wurde.

6. Elektrische Verbinderanordnung (10), umfassend:
einen elektrischen Verbinder (14) nach einem der Ansprüche 1 bis 3; und
einen elektrischer Verbinder (12) nach Anspruch 4 oder 5.

## Revendications

1. Connecteur électrique (14), comprenant :
un logement de connecteur mâle (15) ayant une partie saillante (18) en forme de sablier dimensionnée pour être inséré dans et s'ajuster étroitement au sein d'une cavité en forme de sablier (17) d'un connecteur femelle (12), la partie saillante en forme de sablier comprenant des zones périphériques supérieure (22) et inférieure (24) sur des côtés opposés d'une zone centrale (26), les zones périphériques supérieure et inférieure (24) étant plus larges que la zone centrale (26), le connecteur femelle (12) comportant un premier réseau de broches électroconductrices (30) disposées au sein de la cavité (17) ; et
un second réseau de prises électroconductrices (28), qui sont contenues au sein de la partie saillante (18) et sont dimensionnées et alignées de sorte que lors d'une insertion de la partie saillante (18) dans la cavité (17), chacune des broches (30) est introduite dans et fait un contact électrique avec une respective des prises (28),
dans lequel une première prise du second réseau est positionnée au sein de la zone périphérique supérieure (22),
une deuxième prise du second réseau est positionnée au sein de la zone périphérique inférieure,
**caractérisé en ce qu'**une troisième prise du second réseau est positionnée au sein de la zone centrale (26) ;
la zone périphérique supérieure (22) a une forme rectangulaire et la zone périphérique inférieure (24) a une forme trapézoïdale ;
et **en ce que** le second réseau comprend au moins 100 prises.

2. Connecteur selon la revendication 1, dans lequel les broches sont en retrait à l'intérieur de la cavité, et dans lequel la partie saillante est mise en forme de sorte que les broches ne viennent en contact avec les prises qu'après que la partie saillante a été insérée dans la cavité.

3. Connecteur selon la revendication 1, dans lequel la partie saillante comprend des trous de centrage ayant des chanfreins d'entrée (36) qui sont alignés avec les prises.

4. Connecteur électrique (12), comprenant :
un logement de connecteur femelle (16) définissant une cavité (17) ayant une forme de sablier, dimensionnée pour avoir une partie saillante en forme de sablier (18) d'un logement de connecteur mâle (15) insérée dans celui-ci et s'ajuster étroitement au sein de celui-ci, la cavité (17) en forme de sablier comprenant des zones périphériques supérieure (22) et inférieure (24) sur des côtés opposés d'une zone centrale (26), les zones périphériques supérieure (22) et inférieure (24) étant plus larges que la zone centrale (26), le connecteur mâle comportant un second réseau de prises électroconductrices (28) ; et
un premier réseau de broches électroconductrices (30) disposées au sein de la cavité (17), les broches (30) étant dimensionnées et alignées de sorte que lors d'une insertion de la partie saillante (18) dans la cavité (17), chacune des broches est introduite dans, et fait un contact électrique avec, une respective des prises, dans lequel une première broche (30) du premier réseau est positionnée au sein de la zone périphérique supérieure (22), une deuxième broche du premier réseau est positionnée au sein de la zone périphérique inférieure (24),
**caractérisé en ce qu'**une troisième broche du premier réseau est positionnée au sein de la zone centrale (26) ;
la zone périphérique supérieure (22) a une forme rectangulaire et la zone périphérique inférieure (24) a une forme trapézoïdale ;
et **en ce que** le premier réseau comprend au moins 100 broches.

5. Connecteur selon la revendication 4, dans lequel les broches sont en retrait à l'intérieur de la cavité, moyennant quoi les broches ne viennent en contact avec les prises qu'après que la partie saillante a été insérée dans la cavité.

6. Ensemble connecteur électrique (10), comprenant :
un connecteur électrique (14) selon l'une quelconque des revendications 1 à 3 ; et
un connecteur électrique (12) selon la revendication 4 ou la revendication 5.
